# EUROPÄISCHE PATENTSCHRIFT

(11) **EP 2 680 817 B1**
(45) Veröffentlichungstag und Bekanntmachung des Hinweises auf die Patenterteilung: **25.05.2016**
(21) Anmeldenummer: 12707101.7
(22) Anmeldetag: 05.03.2012
(51) Int. Cl.: A61K 9/00, A61K 47/38, A61K 9/08, A61K 47/36

(54) **VERWENDUNG EINES VISKOELASTISCHEN FLUIDS ZUR HERSTELLUNG EINES MEDIZINPRODUKTES FÜR DIE CHIRURGISCHE BEHANDLUNG DES AUGES**
USE OF A VISCOELASTIC FLUID FOR PRODUCING A MEDICINAL PRODUCT FOR SURGICALLY TREATING THE EYE
UTILISATION D'UN FLUIDE VISCOÉLASTIQUE POUR LA PRÉPARATION D'UN PRODUIT MÉDICAL POUR LE TRAITEMENT CHIRURGICAL DE L'OEIL

(30) Priorität: 03.03.2011 AT 2882011
(43) Veröffentlichungstag der Anmeldung: 08.01.2014
(73) Patentinhaber: VALEANT sp. z o.o. sp. j., 35-959 Rzeszów (PL)
(72) Erfinder: FINDL, Oliver, A-1010 Wien (AT)
(74) Vertreter: Schwarz & Partner
(86) Internationale Anmeldenummer: PCT/EP2012/053710
(87) Internationale Veröffentlichungsnummer: WO 2012/117115

(56) Entgegenhaltungen:
- WO-A2-2009/074853
- ELENA S. NOVITSKAYA, SIMON J. DEAN, ET AL.: "Effects of some ophthalmic medications on pupil size: a literature review", CAN. J. OPHTHALMOL., Bd. 44, Nr. 2, 2009, Seiten 193-197, XP002687425,
- TOMOMI HIGASHIDE, KAZUHISA SUGIYAMA: "Use of viscoelastic substance in ophthalmic surgery - focus on sodium hyaluronate", CLINICAL OPHTHALMOLOGY, Bd. 2, Nr. 1, 2008, Seiten 21-30, XP002687426,
- KESHNER R M: "6 TIPS TO CLEAR CORNEA CATARACT SURGERY", REVIEW OF OPTHALMOLOGY, JOBSON OPTICAL GROUP, UNITED STATES, 1 April 1999 (1999-04-01), pages 120-124, XP003035817, ISSN: 1081-0226
- "HPMC IN BALANCED SALT SOLUTION. INTRAOCULAR INJECTION", MOORFIELDS PHARMACEUTICALS' BROCHURE, 14 May 2009 (2009-05-14), XP055194676, Retrieved from the Internet: URL:http://www.moorfieldspharmaceuticals.c o.uk/uploads/tx_zftablecontents/Moorfields _HPMC_Brochure.pdf [retrieved on 2015-06-10]

## Beschreibung

Die Erfindung betrifft die Verwendung eines viskoelastischen Fluids, wie durch die Patentansprüche defniert, zur Herstellung eines
Medizinproduktes für die chirurgische Behandlung des Auges, welches Fluid nach Applikation auf die Augenoberfläche einen Vergrößerungseffekt der Linse und Pupille bewirkt.
Das viskoelastische Fluid dient zum Schutz der Cornea vor Austrocknung sowie vor Epithelschäden während Augenoperationen, wie Kataraktoperationen, Glaukomoperationen, Fremdkörperentfernung, oder Operationen des hinteren Augenbereichs (Hinterabschnittschirurgie wie z.B. Vitrektomie, Trabekulektomie).
Gewöhnlich wird das Auge während einer Operation in regelmäßigen Abständen mit einer Salzlösung befeuchtet, um ein Austrocknen der Cornea zu verhindern. Dieser Vorgang unterbricht jedoch die Arbeit des Operateurs, beeinträchtigt den operativen Fortgang und zerstört die Homöostase des Tränenfilms. In weiterer Folge werden dadurch wichtige Bestandteile des Tränenfilms wie z.B. entzündungshemmende Enzyme, Lipide, Mucopolysaccharide ausgewaschen.
Es ist bekannt, ein viskoelastisches Fluid zur Befeuchtung des Auges vor einer Kataraktoperation, welche die Cornea während der Operation effizient vor einer Austrocknung schützt, zu verwenden. Das Fluid wird hierbei aus einer Spritze ausgedrückt und falls für die optimale Verteilung notwendig, mit einer Spatula oder Microsponge auf der Cornea verteilt.
In der WO 2009/07853 ist eine opthalmische Zusammensetzung beschrieben, die ein pupillenerweiterndes (mydriatic) Agens und ein viskoelastisches Polymer enthält.
In XP002687425 ist beschrieben, dass die Polymere HPMC und Na-hyaluronat während Augenoperationen im vorderen Augenabschnitt eingesetzt werden können um die Vorderkammer des Auges zu stabilisieren und die Gewebeoberflächen während des Eingriffs zu schützen. Nach dem Eingriff werden die Polymere wieder entfernt.
In XP002687426 wird die Verwendung von Na-hyaluronat in der Augenchirurgie in Form von "OVDs", d.h. von "Devices", die während der Operation ins Auge injiziert werden, beschrieben.

Die XP003035817offenbart ophthalmologische Produkte, wie Goniosol Augentropfen, eine sterile 2,5%ige, viskose HPMC-Zubereitung zur Bedeckung der Cornea, die im Rahmen ophthalmologischer chirurgischer Eingriffe die Augenoberfläche schützt und außerdem einen 1,5 fachen Vergrößerungseffekt hervorruft. Die vorliegende Erfindung stellt sich zur Aufgabe, ein Medizinprodukt mit einem viskoelastischen Fluid bereitzustellen, welches nach Applikation auf das Auge einen Vergrößerungseffekt für Linse und Pupille bewirken soll.
Es hat sich gezeigt, dass ein viskoelastisches Fluid mit folgender Zusammensetzung einen Vergrößerungseffekt der Linse und Pupille imn Ausmaß von 10-15% bewirkt:

Eine offenbarte Formulierung enthält mindestens ein viskositätserhöhendes, physiologisch verträgliches Polymer, das bekannterweise die Augenoberfläche befeuchtet, wie zum Beispiel Hydroxypropylmethylzellulose, Carboxymethylzellulose, Hydroxyethylzellulose, Hyaluronsäure, Natriumalginat, Hydroxylpropyl-Guar Polyvinylpyrrolidon, Polyvinylalkohol, Polymethacrylsäure (Carbomer), Polyoxyethylen-Polyoxypropylen-Copolymer (Poloxamer), Polyethylenglykol
in einer Konzentration von 0,01-30%
oder eine Kombination von zwei oder mehreren dieser Polymere.

Die erfindungsgemäße Zusammensetzung, wie durch die Ansprüche definiert, wird am Auge eingesetzt. Daher weist die
Zusammensetzung vorzugsweise einen pH-Wert von 6 bis 8,5, vorzugsweise 6,5 bis 8, noch mehr bevorzugst 6,8 bis 7,6, auf.
Bei der Verabreichung von Zusammensetzungen am Auge ist es weiter von Vorteil, wenn diese eine der Tränenflüssigkeit vergleichbare Osmolarität aufweisen. Daher beträgt die Osmolarität der erfindungsgemäßen Zusammensetzung vorzugsweise 200 bis 400 mosmol/l, noch mehr bevorzugt 280 bis 330 mosmol/l.
Die dabei benötigten Hilfsstoffe wie z.B. Puffersalze, Stabilisatoren, Hilfsstoffe zur Einstellung der gewünschten Osmolalität und Hilfsstoffe zur Erhöhung der Verträglichkeit hängen jeweils von der Formulierung ab und sind dem Fachmann hinreichend bekannt.

Beispiel für ein nicht erfindungsgemässes viskoelatisches Fluid:
Cornea protect Zusammensetzung:
   - Wasser f. Injektionszwecke
   - Natriumhydroxid
   - Milchsäure 90%
   - Natriumchlorid
   - Kaliumchlorid
   - Calciumchlorid x 2H2O
   - Hydroxypropylmethylzellulose
Füllvolumen: 2 ml
pH-Wert 6,8-7,6
Osmolalität: 265-330 mOsmol/kg

Eine konkrete Formulierung ist wie folgt, wobei sich die Mengen der angegebenen Stoffe auf 1 ml Wasser für Injektionszwecke beziehen:

| | |
|---|---|
| Natriumhydroxid: | 1,15 mg |
| Milchsäure 90%: | 2,40 mg |
| Natriumchlorid: | 6,00 mg |
| Kaliumchlorid: | 0,40 mg |
| Calciumchloridx2H₂O: | 0,27 mg |
| Hydroxypropylmethylcellulose: | 22,00 mg |

Statt Hydroxypropylmethylcellulose kann auch bevorzugt Hyaluronsäure in einer Menge im Bereich von 0,01-10%, insbesondere 15,4 mg/ml, vorhanden sein. In diesem Fall können ferner vorhanden sein:

| | |
|---|---|
| Natriumchlorid: | 8,15 mg/ml |
| Di-Natriumhydrogenphosphatdodecahydrat: | 0,70 mg/ml |
| Natriumdihydrogenphosphatdihydrat: | 0,056 mg/ml |

Der pH-Wert ist vorzugsweise im Bereich von 6,8-7,6 und die Osmolarität zwischen 280-330. Im folgenden Beispiel wird der optische Vergrößerungseffekt an der Linse und Pupille an einem künstlichen Auge gezeigt.

### Beispiel

2 ml des oben beschriebenen viskoelastischen Fluids wurden an einem Modellauge appliziert. Im Vergleich zum unbehandelten Modellauge betrug der optische Vergrößerungseffekt ca. 10%.
Das viskoelastische Fluid kann in einem in einer Schutzhülle eingeschweißten Behältnisses enthalten sein, das als Medizinprodukt in der Augenchirurgie verwendet wird. Die Erfindung betrifft ferner das in der Schutzhülle eingeschweißte Behältnis.
Das Behältnis ist derart gestaltet, dass das Fluid über eine Sollbruchstelle dem Behältnis entnommen werden kann, wobei das Herstellungsverfahren durch die Kombination der Maßnahmen gekennzeichnet ist, dass
- das erfindungsgemäß verwendete viskoelastische Fluid in das Behältnis abgefüllt wird, wonach dieses verschlossen wird,
- das verschlossene Behältnis in einer Schutzhülle eingeschweißt wird, wonach
- das eingeschweißte Behältnis samt Schutzhülle einer thermischen Sterilisation unterzogen wird.

Nach dem Einschweißen des Behältnisses in die Schutzhülle wird durch die terminale Sterilisation des Produktes die innere und äußere Sterilität des Produktes gewährleistet. Ein weiterer Vorteil des erfindungsgemäßen Verfahrens besteht darin, dass dem viskoelastischen Fluid kein Konservierungsmittel zugegeben werden braucht.

Das erfindungsgemäß hergestellte Behältnis gewährleistet einen höheren Komfort für den Chirurgen bei der Verwendung, sowie eine höhere Sicherheit beim Patienten.

Eine bevorzugte Ausführungsform des erfindungsgemäßen Verfahrens besteht darin, dass das Behältnis ein Einzeldosenbehältnis ist.

Das Behältnis bzw. Einzeldosenbehältnis ist bevorzugt aus Polypropylen oder Mischungen von Polyethylen oder Polypropylen mit Copolymeren aus Ethylen und Propylen oder aus einem Laminat.

Die Schutzhülle besteht vorzugsweise aus einem sterilisierbaren Medizinalpapier und einer Verbundfolie (z.B. Medipeel® Pouch von Sengewald) oder **Tyvek**^{®} Material (Hersteller DuPont).

Die thermische Sterilisation kann bei einer Temperatur zwischen 80 und 140°C vorgenommen werden.

Die Erfindung betrifft ferner das nach dem erfindungsgemäßen Verfahren herstellbare, in einer Schutzhülle eingeschweißte Behältnis als solches.

Die Einzeldosenbehältnisse bestehen vorzugsweise aus Polypropylen (PP) in pharmazeutischer Qualität. Der für die Herstellung der Einzeldosenbehältnisse bevorzugt verwendete Polypropylen Rohstoff weist folgende Eigenschaften auf:
Schmelzpunkt (bestimmt nach ISO 3146): 100°C - 260°C
Vicat-Erweichungstemperatur (10N, 50°C pro Stunde; bestimmt nach ISO 306): 80°C - 240°C;
Schmelzflussindex (230°C / 2,16kg; bestimmt nach ISO 1133): 0,1g/10 min - 50g/10 min;
Streckdehnung (50mm/min; bestimmt nach ISO 527-2): 1% - 30%;
Charpy-Kerbschlagzähigkeit (bei 23°C; bestimmt nach ISO 179): 1 kj/m² - 20 kj/m²

Die Schutzhülle für das Einzeldosenbehältnis besteht vorzugsweise aus einem sterilisierbaren Medizinalpapier und einer Spezialverbundfolie, wobei eine Seite durchsichtig ist (z.B. Medipeel^{®}Pouch von Sengewald) und gewährleistet äußere Sterilität des Einmaldosenbehältnisses.

### Beschreibung der Sterilisation

Durch die spezielle Verpackung des viskoelastischen Fluides in einem Einzeldosenbehältnis und einer darüberliegenden Schutzhülle wird somit in einem einzigen - terminalen - Sterilisationsschritt die innere und äußere Sterilität des Produktes gewährleistet. Die bevorzugte Art der Sterilisation ist die physikalische Sterilisation durch Hitze in einem Temperaturbereich von 80°C-140°C z.B. durch Heisswasserberieselung, Sattdampfsterilisation oder Sterilisation mit einem Dampf-Luft-Gemisch.

Es hat sich überraschend gezeigt, dass die terminale Sterilisation mit ionisierenden Strahlen im vorliegenden Fall nicht geeignet ist, da es zum unkontrollierten Abbau der viskositätserhöhenden Polymere im Fluid kommt und das Produkt nach der Sterilisation somit die erforderlichen viskoelastischen Eigenschaften nicht mehr aufweist. Alle anderen Sterilisationsmethoden haben den Nachteil, dass die Sterilisation nicht als terminale Sterilisation im Endbehältnis erfolgen kann, sondern zwei Sterilisationsschritte für innere and äußere Sterilität erforderlich wären.

## Patentansprüche

1. Wässeriges viskoelastisches Fluid, welches
a) Hyaluronsäure in einer Konzentration von 0.01% bis 30% umfasst,
b) einen pH-Wert von 6 bis 8,5 aufweist und
c) eine Osmolarität von 200 bis 400 mosmol/l aufweist,
zur Anwendung als Medizinprodukt für die chirurgische Behandlung des Auges, welches Fluid nach Applikation auf die Augenoberfläche aus einem Einmaldosenbehältnis einen optischen
Vergrösserungseffekt der Linse und Pupille bewirkt.

2. Viskoelastisches Fluid zur Anwendung als Medizinprodukt nach Anspruch 1, **gekennzeichnet durch** einen pH-Wert von 6,5 bis 8.

3. Viskoelastisches Fluid zur Anwendung als Medizinprodukt nach Anspruch 1, **gekennzeichnet durch** einen pH-Wert von 6,8 bis 7,6.

4. Viskoelastisches Fluid zur Anwendung als Medizinprodukt nach einem der Ansprüche 1 bis 3, **gekennzeichnet durch** einen Osmolarität von 280 bis 330 mosmol/l.

5. Viskoelastisches Fluid zur Anwendung als Medizinprodukt nach einem der Ansprüche 1 bis 4, **gekennzeichnet durch** Hyaluronsäure in einer Konzentration von 0.01% bis 10%.

## Claims

1. An aqueous viscoelastic fluid which
a) comprises hyaluronic acid at a concentration of 0.01-30%;
b) has a pH value ranging from 6 to 8.5; and
c) has an osmolarity ranging from 200 to 400 mosmol/l; for use as medicinal product for the surgical treatment of the eye, which fluid after application onto the surface of the eye from a single-dose receptacle produces an optical magnifying effect of the lens and the pupil.

2. Viscoelastic fluid for use as medicinal product according to claim 1, **characterized by** a pH value ranging from 6.5 to 8.

3. Viscoelastic fluid for use as medicinal product according to claim 1, **characterized by** a pH value ranging from 6.8 to 7.6.

4. Viscoelastic fluid for use according to one of claims 1 to 3, **characterized by** an osmolarity ranging from 280 to 330 mosmol/l.

5. An aqueous viscoelastic fluid for use as medicinal product according to one of claims 1 to 4, **characterized by** hyaluronic acid at a concentration of 0.01-10%.

## Revendications

1. Fluide viscoélastique aqueux,
a) qui inclut de l'acide hyaluronique dans une concentration de 0,01 % à 30 %,
b) présente un pH d'une valeur de 6 à 8,5, et
c) présente une osmolarité de 200 à 400 mosmol/l,
pour l'application à titre de produit médicinal pour le traitement chiuurgical de l'oeil,
ledit fluide provoquant, après application sur la surface de l'oeil à partir d'un récipient d'une dose à usage unique, un effet d'agrandissement optique de la lentille et de la pupille.

2. Fluide viscoélastique pour l'application à titre de produit médicinal selon la revendication 1, **caractérisé par** un pH d'une valeur de 6,5 à 8.

3. Fluide viscoélastique pour l'application à titre de produit médicinal selon la revendication 1, **caractérisé par** un pH d'une valeur de 6,8 à 7,6.

4. Fluide viscoélastique pour l'application à titre de produit médicinal selon l'une des revendications 1 à 3, **caractérisé par** une osmolarité de 280 à 330 mosmol/l.

5. Fluide viscoélastique pour l'application à titre de produit médicinal selon l'une des revendications 1 à 4, **caractérisé par** de l'acide hyaluronique dans une concentration de 0,01 % à 10 %.
